(19) **Europäisches Patentamt**
**European Patent Office**
**Office européen des brevets**

(11) **EP 4 517 768 A1**

(12) **EUROPEAN PATENT APPLICATION**

(43) Date of publication:
05.03.2025 Bulletin 2025/10

(21) Application number: 23195163.3

(22) Date of filing: 04.09.2023

(51) International Patent Classification (IPC):
**G16H 30/00** (2018.01)   **G06T 7/00** (2017.01)
**G16H 50/20** (2018.01)

(52) Cooperative Patent Classification (CPC):
**G16H 50/20; G06T 7/0012; G16H 30/00**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Siemens Healthineers AG**
**91301 Forchheim (DE)**

(72) Inventors:
• **Chekkoury, Andrei**
**91054 Erlangen (DE)**
• **Eibenberger, Eva**
**90427 Nürnberg (DE)**
• **Gibson, Eli**
**Plainsboro, 08536 (US)**
• **Soza, Grzegorz**
**90562 Heroldsberg (DE)**

(74) Representative: **Siemens Healthineers**
**Patent Attorneys**
**Postfach 22 16 34**
**80506 München (DE)**

(54) **COMPUTER-IMPLEMENTED METHOD FOR CLINICAL DECISION SUPPORT, CLINICAL DECISION SUPPORT SYSTEM, COMPUTER PROGRAM AND ELECTRONICALLY READABLE STORAGE MEDIUM**

(57) Computer-implemented method for clinical decision support, wherein a trained first function (34) determines first output data (35) regarding at least one clinical condition of a patient from first input data (33) comprising image data of the patient, the first output data (35) comprising at least one confidence value associated with or as result information, wherein the confidence value is modified by a second function (47) based on case-specific background information (37) regarding the patient and/or the acquisition of the image data, the case-specific background information (37) provided as second input data to the second function (47).

FIG 2

**Description**

The present invention is in the field of medical imaging, which is used for of creating visual representations and analysis of the interior of a body for clinical analysis and medical intervention, as well as visual representation and analysis of the function of some organs or tissues.

The invention concerns a computer-implemented method for clinical decision support, wherein a trained first function determines first output data regarding at least one clinical condition of a patient from first input data comprising image data of the patient, the first output data comprising at least one confidence value associated with or as result information. The invention further concerns a clinical decision support system, a computer program and an electronically readable storage medium.

The image data of a patient can be obtained from a medical imaging modality such as Computed Tomography (CT), ultrasound inaging (US), radiography, fluoroscopy or magnetic resonance imaging (MR).

The image data is obtained by performing a scan of the patient or a part of the patient's anatomy with the medical imaging modality. A scan protocol defines the scan parameters of the imaging modality. In some cases, image raw data is obtained from the imaging modality, such raw data may be reconstructed to obtain reconstructed image data.

The image data of the patient may comprise a raw data set, a reconstructed image data set, and/or an image data set which has been further processed after reconstruction.

Machine learning has been widely employed in clinical evaluation tasks, in particular in clinical decision supports. For example, trained functions, which have been trained by machine learning, may be used to evaluate image data of patients. In general, a trained function mimics cognitive functions that humans associate with other human minds. In particular, by training based on training data the trained function is able to adapt to new circumstances and to detect and extrapolate patterns.

For example, a trained function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network (CNN) or a convolutional deep neural network. Furthermore, a neural network can be an adversarial network, a deep adversarial network and/or a generative adversarial network (GAN).

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

Regarding the evaluation of image data in clinical decision support, for example in detecting and classifying lesions, often neural networks, in particular CNNs, are employed. The corresponding functions are trained using supervised learning on large training data bases.

Trained functions, which have been trained by supervised learning, are data driven and, in general, do not incorporate prior knowledge. They rely on learning patterns from the training data they are confronted with during the training steps. Good results are achieved, in particular, when large amounts of training data are used and the training data are diverse. However, trivial errors can occur in the output data, in particular when the input data describes a scenario which has not been used in the training data. To address this problem, it has been proposed to add confidence values (confidence scores) to the output data of trained functions. For example, confidence values can be associated with result information of the output data. Such result information may, for example, be a classification information.

In a concrete example, in binary classification for means of triage, the computation of confidence values allows grouping of artificial intelligence results into high-confidence positive cases, high-confidence negative cases and low confidence findings. However, due to lack of internal knowledge, these confidence scores may, at least in some cases, be of lower reliability themselves, in particular regarding scenarios not trained or edge cases.

Challenging cases, which may result in false-positives and false-negative matches, are, according to the state of the art, addressed by manual addition of annotated cases into the training pool. This is a slow and cost-intensive process that involves incremental algorithm updates, generally triggered by unsatisfactory algorithm performance reported by end-users.

An article by Weijie Chen et al., "Calibration of medical diagnostic classifier scores to the probability of disease", Stat Methods Med Res. 27 (2018), pages 1394-1409, investigates three methods for calibrating classifier scores to the probability of disease scale and develops uncertainty estimation techniques for these methods. It is shown that calibration without affecting the discrimination performance can be achieved.

According to US 2020/0320354 A1, medical images may be classified by receiving a first medical image. The medical image may be applied to a machine learned classifier. A label of the medical image and a measure of uncertainty may be generated. The measure of uncertainty may be compared to a threshold. The first medical image and the label may be output when the measure of uncertainty is within the threshold.

US 2022/0293247 A1 discloses systems and methods for performing a medical imaging analysis task for making a clinical decision. One or more input medical images of a patient are received and a medical imaging analysis task is performed from the one or more input medical images using a machine learning based network. The machine learning based network generates a probability score associated with the medical imaging analysis task. An uncertainty measure associated with the probability score is determined. A clinical decision is made based on the probability score and the uncertainty measure.

A further method regarding the use of trained functions in clinical decision support is disclosed in US 2022/0309667 A1. Here, systems and methods for assessing expansion of an abnormality are provided. Based on a first input medical image of a patient depicting an abnormality at a first time and a second input medical image of the patient depicting the abnormality at a second time, features are extracted and the expansion of the abnormality is assessed based on the extracted features using a trained machine learning based network.

It is an object of the current invention to further improve the quality and/or interpretability of result information of trained functions in clinical decision making.

This object is achieved by providing a computer-implemented method, a clinical decision support system, a computer program, and an electronically readable storage medium according to the independent claims. Advantageous embodiments are described in the dependent claims.

In a computer-implemented method as initially described, according to the invention, the confidence value is modified by a second function based on case-specific background information regarding the patient and/or the acquisition of the image data, the case-specific background information being provided as second input data to the second function.

The trained first function, which may be an image evaluation function, may preferably comprise at least one neural network, in particular a CNN. Hence, the trained first function may in particular be a machine learning-based network, as in principle known from the art. In embodiments, the trained first function may be a classifier and/or the result information may be a classification. In triage or other applications, the classifier may preferably be a binary classifier.

The confidence value may be the basis for the result information. For example, in a classifier network as the trained first function, the output may be a probability for each class. This probability is the confidence value (in this case sometimes also called classification scores). Hence, in a binary classifier, usually one confidence value is used. Generally, there may be more than one confidence value, of which as least one can modified by the second function. Thresholds may be used to derive the result information from the at least one confidence value. For example, in binary classification, a result information may be determined as "positive" if the confidence value is equal to or higher than a threshold, for example 67 %.

The confidence value may be used to group into high-confidence positive cases, high-confidence negative cases and low confidence findings.

As a consequence, in general, the modification of the confidence value may also lead to a modification of the result information. For example, if a confidence value falls below or rises above a threshold by modification, the result can be changed. Hence, the result information may be modified based on the modified confidence score. However, in embodiments, at least one confidence value may also be an additional uncertainty measure associated with a result, for example a score. Generally, both the result information and the modified confidence score may be output as a last step of the method.

The methods and systems described here are for clinical decision support, meaning that any diagnostical, therapeutical or other clinical decisions are made at a later point in time by a user, in particular a physician, who may take the result information and the modified confidence values (confidence scores) into account accordingly. Hence, the methods and systems describe here neither comprise diagnosis nor therapy.

The case-specific background information may comprise or describe prior knowledge, but may also at least partly be determined taking the first output data into account. In particular, the case-specific background information may describe circumstances of the case (to which the first input data relates) which hint at or describe how well the trained first function is expected to perform or to have performed and/or allow a plausibility check. Concrete examples will be described below.

A main idea and advantage of the invention are to not modify or adapt the trained first function to take case-specific background information into account, but to use the case-specific background information to modify the confidence value to reflect the additional knowledge. That is, the trained first function may be fixed (in particular locked) and/or pre-trained unconditioned regarding the background information. In other words, at least one background information is used only to modify the confidence value. The at least one background information is not used to modify or adapt the trained first function. There may be a-priori prior information the trained first function is partially conditioned to, which is not comprised by the case-specific background information.

It is proposed to solve the problem of providing reliable and/or higher-quality confidence values by incorporating information in the calculation of the confidence values in independent steps, namely first applying the trained first function, which may be considered a fixed, first model, yielding at least one associated confidence value associated with or as result information. After the first output data has been determined, the second function is applied, which may be considered a second, conditioning model. For example, if a particular case is considered by the fixed model to be a positive/negative match, prior information, such as whether this case belongs to a known class of edge cases or cases for which a weakness of the trained first function is known, is assessed by the second function, and the case is re-evaluated according to this background information. This may lead to a reduced number of false-positives or false-negatives, in particular when the final result information is determined based on the modified confidence values, and/or to a better calibrated confidence value.

The first output data and the confidence value may be provided via an interface to a data carrier or to a display for a user.

The first output data and the confidence value may be used for clinical decision support or to store or display an information related to clinical decision support.

The confidence value may be used to classify the first output data as one of: high-confidence positive case, high-confidence negative case and low confidence finding.

Based on the confidence value, a further method step may be carried out automatically or proposed to a user. A further method step can, e.g., comprise one of:

- Applying an image processing step.
- Notifying that a further scan of the patient is required,
- Selecting a scan protocol for a further scan of the patient
- Proposing or displaying a scan protocol selection for a further scan of the patient to a user.

An image processing step can, e.g., comprise one of:

- an image reconstruction step to obtain reconstructed image data from raw data,
- an image processing step which affects image parameters such as noise, contrast, brightness, sharpness, or other,
- an image analysis step, such as image segmentation, identification of an image feature, in particular an image feature which indicates an anatomical or pathological feature, or quantification of an image parameter, material decomposition to identify base materials in the image data.

The first function, which may be high-dimensional, can still be trained unconditioned on case-specific background information, in particular prior knowledge, or partially conditioned on a priori prior information, which is not included in the case-specific background information assessed by the second function. The trained first function can then be held fixed. This is, in particular, important wherever complicated and time-consuming admission procedures are required for every updated version. A second function, which may use the output of the trained first function as well as the case-specific background information as second input data, is added to generate a conditioned confidence value. The specification of the case-specific background information is not required for the development of the (fixed) trained first function, but may be defined at later development stage.

Here, the first and the second functions are independent in the sense that they do not interact and are, in particular, executed in a strictly sequentially temporal order. In other words, incorporating case-specific background information is performed using an independent, in particular low-dimensional, conditioning model, that is, the second function, which may be developed and updated more easily.

In summary, it is proposed to integrate case-specific background information in the decision-making mechanism, in particular of machine learning, by combining the case-specific background information with confidence values (confidence scores) of trained first functions. By this combination, the performance of already trained first functions is improved without the need to modify the first function or its regularization parameters.

In embodiments, the case-specific background information is at least partly derived from first output data, in particular regarding a feature, in particular a lesion, in the image data. For example, regarding a plausibility check, the geometrical shape of a lesion may be relevant case-specific background information. Candidates for lesions may already be pre-segmented, but a lesion can only be chosen once the feature has been identified as relevant by the trained first function.

It is generally noted that, regarding lesions or generally features of interest, trained first functions often also output their location in the first output data, which is useful regarding determining and/or interpreting the background information.

In concrete embodiments, the case-specific background information may be chosen from the group comprising

- anatomical information, in particular an anatomical segmentation of the image data;
- shape information describing the geometrical shape of a feature, in particular a lesion, comprised in the first output data;
- metadata information associated with the first input data; and
- additional clinical information regarding the patient.

In preferred embodiments, the anatomical location of a pathology may be considered case-specific background information, which may be derived from first output data and/or the image data. Anatomical information may, preferably additionally, also comprise segmentation information, in particular allowing to semantically identify anatomical features in the image data. For example, a plausibility check may be performed regarding a lesion which has been identified as a pathology of a certain anatomical feature, wherein the anatomical location of the lesion is used to check which anatomical feature in in this location according to segmentation information. If the anatomical features do not match, the confidence value may be lowered. For example, the confidence score of a first function trained to detect liver lesions may be lowered if the flagged lesion is located in the left lower abdominal quadrant. In this manner, a false positive finding can be corrected by the second function.

Additionally or alternatively, the case-specific background information may also comprise shape information describing the geometrical shape of a feature, particular a lesion, comprised in the first output data. In preferred embodiments, the geometric shape of a finding can be considered case-specific background information. In concrete examples, the confidence score of a first function trained to detect intraventricular brain hemorrhages shall be lowered if the flagged finding, i.e., feature, has clear crescent shape, typical for a subdural hematoma.

Regarding metadata information, this may preferably comprise metadata relating to the patient and/or metadata relating to the acquisition of the image data. Such metadata are usually comprised in or associated with an image data file, which may, for example, be in the DICOM standard. In general, it is a known fact that demographics of the patient as well as acquisition parameters regarding the image data, for example, when using a CT as imaging modality, slice thickness, slice spacing and used reconstruction kernels, play an important role in the performance of the human reader, as well as in the performance of trained functions. In preferred embodiments, metadata information can be used as case-specific background information to play a role in determining, in this case modifying, the confidence value of first output data. For example, the confidence score of finding can be influenced by the image acquisition and the reconstruction parameters of the image data being processed. In a concrete example, higher slice thickness may lead to a lower confidence value due to information loss, such that, for example, the probability for a bleeding may be reduced from 90% to 80% due to slice thickness.

In embodiments, the additional clinical information may be chosen from the group comprising

- at least one laboratory result;
- at least one physiological parameter of the patient;
- the presence of at least one inflammatory marker;
- at least one score, in particular a risk score and/or a clinical score, determined for the patient; and
- anamnesis information of the patient.

Hence, several other clinical factors, which are generally already available at the time of the imaging scan, can be used as case-specific background information. For example, laboratory values, physiological parameters (for example blood pressure, body temperature, breathing rate, heart rate, and the like), inflammatory markers, or an already assessed Glasgow Coma Scale of the patient might influence the confidence value of the trained first function. Additional information extracted from the patient's anamnesis like presentation reason, prior surgery, presence of implanted devices, and the like can as well be taken into consideration as case-specific prior information that a human reader generally accounts for, but a trained first function does not.

In concrete embodiments, the additional clinical information, in particular the anamnesis information, may at least partly be determined from an electronic patient record of the patient and/or at least one patient registration information. For example, the clinical decision support system executing the method may be connected to an information system, for example a hospital information system (HIS) and/or a radiology information system (RIS), where an electronic patient record and/or patient registration information can be stored. It is noted that generally, if a case-specific background information is missing, a user may also be prompted to supply the missing information.

In preferred embodiments, the case-specific background information may at least partly be provided by at least one trained third function using, as third input data, at least a part of the first input data, in particular the image data. Such a trained third function can, for example, be a segmentation function, a determination function determining a score, and the like. Hence, other artificial intelligence models may also be used to provide case-specific background information. For example, trained functions able to segment and label anatomical features in image data have already been proposed and may also be used in the current invention to determine anatomical information. For example, regarding brain image data evaluation, a trained third function can be used to segment the main brain lobes. In an exemplary embodiment, the segmentation information may be combined with a trained first function identifying critical pathologies, for example infarcts or hemorrhages, to improve the confidence scores, in particular using a plausibility check, and implicitly the performance of the trained first function. Preferably, the at least one trained third function is, in particular also, fixed/locked.

In especially preferred embodiments, the case-specific background information may comprise at least one classification of the case based on development and/or producer-internal evaluation information of the trained first function. Trained functions, that is artificial intelligence models, like human readers, have weaknesses regarding evaluations of challenging pathologies. These weaknesses are often identified during development and internal evaluations, particular at the producer/developer of the trained first function. Such development and/or internal knowledge may also be applied in the second function, when case-specific background information identifies the scenario as matching such a weakness of the first trained function. In this manner, confidence values can be advantageously adjusted. For example, the confidence value generated by the trained first function may be lowered by including knowledge that the trained first function is less performant regarding findings located in specific anatomical location. The case may then be classified as, for example, concerning this specific anatomical location, and the confidence value may be modified accordingly. As a concrete example, it has been observed that brain hemorrhage detection functions may have weaknesses in assessing possible bleedings located along the tentorium or along the falx cerebri, which may sometimes be flagged as false positives.

In preferred embodiments, the second function may comprise at least one of

- performing at least one plausibility check, wherein the confidence value is lowered when an implausibility is detected; and
- lowering the confidence value if, according to the case-specific background information, the case is identified as an edge case and/or relating to a weakness of the trained first function.

As already discussed, a plausibility check may, for example, comprise correlating the anatomical location of a finding with segmentation result (provided as anatomical information) and/or correlating the geometrical shape to known shapes of findings in the respective class. While weaknesses have also already been discussed, edge cases may be rare occurrences not comprised in training data and/or other extreme scenarios, in which the confidence value may also be lowered.

Generally speaking, the second function may comprise at least one non-AI algorithm and/or at least one trained conditioning function. The conditioning model represented by the second function may, on the one hand, include engineered features based on clinical knowledge. However, on the other hand, artificial intelligence may also be employed regarding the second function, which may in particular be a trained second function. In particular, at least a part of the case-specific background information may be used as input data for at least one trained conditioning function, which is preferably low-dimensional. In embodiments, the trained conditioning function may have a lower dimensionality as the trained first function and/or comprise a support vector machine and/or a random forest and/or a regression model. Trained conditioning functions may be employed in any case where the correlation between the background information and modification of the confidence value is more complicated.

Generally speaking, the second input data may also comprise the at least one confidence value of the first output data, which is to be modified. In some embodiments, the second input data may, at least in part, also comprise further first output data. The second function may be, as a whole, structured as a conditioning model, providing the modified confidence value directly as second output data. However, in embodiments, the second function may also comprise

- determining parameters parametrizing a functional correlation between the confidence value of the first output data and the modified confidence value, and
- determining the modified confidence value by applying the functional correlation parametrized by the parameters to the confidence value of the first output data.

Here, the part of the second function yielding the parameters may be understood as a model-structured part of the second function, while the functional correlation is pre-defined. For example, the functional correlation may comprise at least one integral spline. In other words, the preferably low-dimensional trained conditioning function may use case-specific background information as input and generate a parametric monotonic confidence value calibration function, such as integral splines, such that the confidence value of the trained first function is mapped to the new, modified confidence value.

The invention also concerns a clinical decision support system, comprising

- a first interface for receiving first input data, the first input data comprising image data of a patient,
- an analysis unit for applying a trained first function to the first input data to determine first output data regarding at least one clinical condition of a patient, the first output data comprising at least one confidence value associated with or as result information,
- a compilation unit for compiling case-specific background information regarding the patient and/or the acquisition of the image data,
- a modification unit for modifying the confidence value by a second function based on the case-specific background information, the case-specific background information being provided as second input data to the second function, and

- an output interface for outputting the modified confidence value.

All features regarding the method according to the invention may analogously be applied regarding the clinical decision support system according to the invention, and vice versa. Hence, the same advantages can be achieved. Any features regarding the method according to the invention may be implemented as modules or functional units of the clinical decision support system according to the invention, and vice versa.

The clinical decision support system may comprise at least one processor and at least one memory. Functional units performing steps of the method according to the invention can be implemented using hardware and/or software. In particular, the clinical decision support system may be configured to execute the steps of the method according to the invention.

In embodiments, the compilation unit may be connected to a second interface for receiving at least a part of the case-specific background information and/or basic information used for determining the case-specific background information. In particular, the clinical decision support system may comprise at least one determination unit for determining at least a part of the case-specific background information from first input data and/or basic information, in particular using a trained third function.

A computer program according to the invention comprises program means such that, when the computer program is executed in a clinical decision support system, it causes the clinical decision support system to perform the steps of method according to the invention. The computer program may be stored on an electronically readable storage medium according to the invention, which thus comprises control information comprising a computer program according to the invention and adapted to, when the electronically readable storage medium is used in a clinical decision support system, configure the clinical decision support system to be able to perform the steps of a method according to the invention. The electronically readable storage medium may preferably be non-transient.

Other objects and features of the present invention will become apparent from the following detailed description considered in conjunction with the accompanying drawings. The drawings, however, are only principle sketches designed solely for the purpose of illustration and do not limit the invention. The drawings show:

Fig. 1    an embodiment of an artificial neural network,

Fig. 2    an embodiment of a convolutional neural network,

Fig. 3    a flowchart of an embodiment of a method according to the invention,

Fig. 4    the principal drawing showing the data and interaction used in the method according to the invention, and

Fig. 5    the functional structure of a clinical decision support system according to the invention.

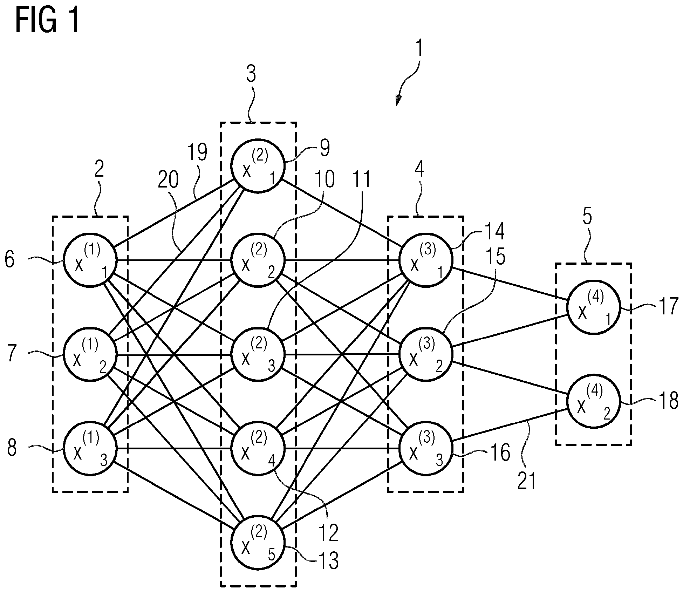

Fig. 1 displays an embodiment of an artificial neural network 1. Alternative terms for "artificial neural network" are "neural network", "artificial neural net" or "neural net".

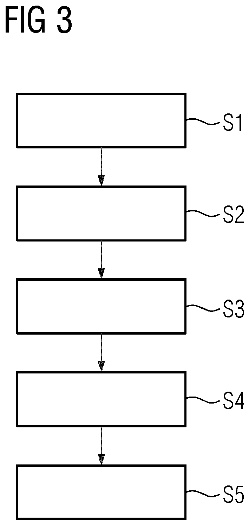

The artificial neural network 1 comprises nodes 6 - 18 and edges 19 - 21, wherein each edge 19 - 21 is a directed connection from a first node 6 - 18 to a second node 6 - 18. In general, the first node 6 - 18 and the second node 6 - 18 are different nodes 6 - 18. It is also possible that the first node 6 - 18 and the second node 6 - 18 are identical. For example, in Fig. 3 the edge 19 is a directed connection from the node 6 to the node 9, and the edge 20 is a directed connection from the node 7 to the node 9. An edge 19 - 21 from a first node 6 - 18 to a second node 6 - 18 is also denoted as "ingoing edge" for the second node 6 - 18 and as "outgoing edge" for the first node 6 - 18.

In this embodiment, the nodes 6 - 18 of the artificial neural network 1 can be arranged in layers 2 - 5, wherein the layers 2 - 5 can comprise an intrinsic order introduced by the edges 19 - 21 between the nodes 6 - 18. In particular, edges 19 - 21 can exist only between neighboring layers of nodes 6 - 18. In the displayed embodiment, there is an input layer 2 comprising only nodes 6 - 8 without an incoming edge, an output layer 5 comprising only nodes 17, 18 without outgoing edges, and hidden layers 3, 4 in-between the input layer 2 and the output layer 5. In general, the number of hidden layers 3, 4 can be chosen arbitrarily. The number of nodes 6 - 8 within the input layer 2 usually relates to the number of input values of the neural network, and the number of nodes 17, 18 within the output layer 5 usually relates to the number of output values of the neural network.

In particular, a (real) number can be assigned as a value to every node 6 - 18 of the neural network 1. Here, $x^{(n)}_i$ denotes the value of the i-th node 6 - 18 of the n-th layer 2 - 5. The values of the nodes 6 - 8 of the input layer 2 are equivalent to the input values of the neural network 1, the values of the nodes 17, 18 of the output layer 5 are equivalent to the output values of the neural network 1. Furthermore, each edge 19 - 21 can comprise a weight being a real number, in particular, the weight is a real number within the interval [-1, 1] or within the interval [0, 1]. Here, $w^{(m,n)}_{i,j}$ denotes the weight of the edge between the i-th node 6 - 18 of the m-th layer 2 - 5 and the j-th node 6 - 18 of the n-th layer 2 - 5. Furthermore, the abbreviation

$w^{(n)}_{i,j}$ is defined for the weight $w^{(n,n+1)}_{i,j}$.

In particular, to calculate the output values of the neural network 1, the input values are propagated through the neural network 1. In particular, the values of the nodes 6 - 18 of the (n+1)-th layer 2 - 5 can be calculated based on the values of the nodes 6 - 18 of the n-th layer 2 - 5 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network 1, wherein values of the input layer 2 are given by the input of the neural network 1, wherein values of the first hidden layer 3 can be calculated based on the values of the input layer 2 of the neural network 1, wherein values of the second hidden layer 4 can be calculated based in the values of the first hidden layer 3, etc.

In order to set the values $w^{(m,n)}_{i,j}$ for the edges 19 - 21, the neural network 1 has to be trained using training data. In particular, training data comprises training input data and training output data (denoted as $t_i$). For a training step, the neural network 1 is applied to the training input data to generate calculated output data. In particular, the training data and the calculated output data comprise a number of values, said number being equal to the number of nodes 17, 18 of the output layer 5.

In particular, a comparison between the calculated output data and the training data is used to recursively adapt the weights within the neural network 1 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer 5, and

$$\delta^{(n)}_j = \left(x^{(n+1)}_k - t^{(n+1)}_j\right) \cdot f'\left(\sum_i x^{(n)}_i \cdot w^{(n)}_{i,j}\right)$$

if the (n+1)-th layer is the output layer 5, wherein f' is the first derivative of the activation function, and $y^{(n+1)}_j$ is the comparison training value for the j-th node of the output layer 5.

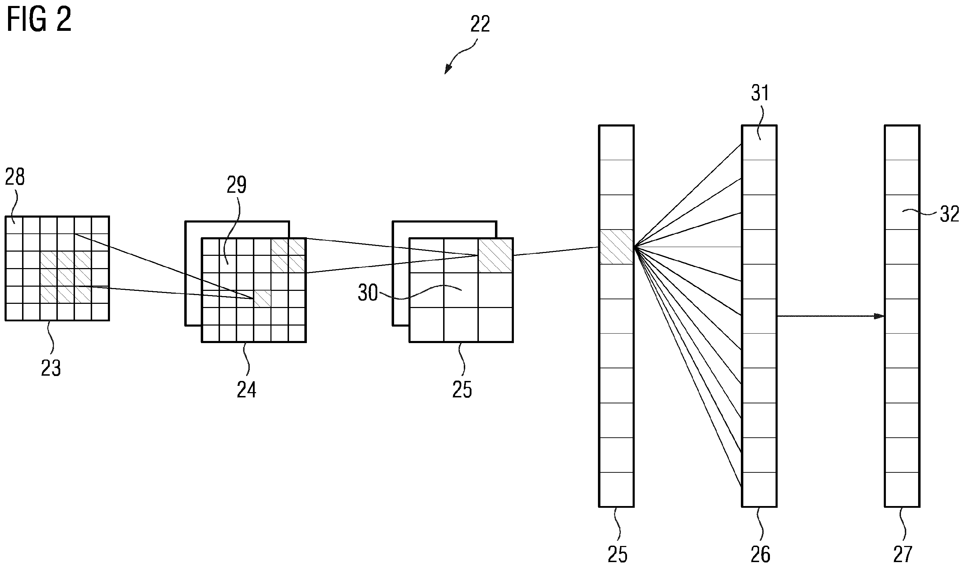

Fig. 2 displays an embodiment of a convolutional neural network 22. In the displayed embodiment, the convolutional neural network 22 comprises an input layer 23, a convolutional layer 24, a pooling layer 25, a fully connected layer 26 and an output layer 27. Alternatively, the convolutional neural network 22 can comprise several convolutional layers 24, several pooling layers 25 and several fully connected layers 26 as well as other types of layers. The order of the layers can be chosen arbitrarily, usually fully connected layers 26 are used as the last layers before the output layer 27.

In particular, within a convolutional neural network 22 the nodes 28 - 32 of one layer 23 - 27 can be considered to be arranged as a d-dimensional matrix or as a d-dimensional image. In particular, in the two-dimensional case the value of the node 28 - 32 indexed with i and j in the n-th layer 23 - 27 can be denoted as $x^{(n)}[i,j]$. However, the arrangement of the nodes 28 - 32 of one layer 23 - 27 does not have an effect on the calculations executed within the convolutional neural network 22 as such, since these are given solely by the structure and the weights of the edges.

In particular, a convolutional layer 24 is characterized by the structure and the weights of the incoming edges forming a convolution operation based on a certain number of kernels. In particular, the structure and the weights of the incoming edges are chosen such that the values $x^{(n)}_k$ of the nodes 29 of the convolutional layer 24 are calculated as a convolution $x^{(n)}_k = K_k * x^{(n-1)}$ based on the values $x^{(n-1)}$ of the nodes 28 of the preceding layer 23, where the convolution * is defined in the two-dimensional case as

$$x_k^{(n)}[i,j] = (K_k * x^{(n-1)})[i,j] = \sum_{i'}\sum_{j'} K_k[i',j'] \cdot x^{(n-1)}[i-i', j-j'].$$

Here the k-th kernel $K_k$ is a d-dimensional matrix (in this embodiment a two-dimensional matrix), which is usually small compared to the number of nodes 28 - 32 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 28 - 32 in the respective layer 23 - 27. In particular, for a convolutional layer 24 the number of nodes 29 in the convolutional layer is equivalent to the number of nodes 28 in the preceding layer 23 multiplied with the number of kernels.

If the nodes 28 of the preceding layer 23 are arranged as a d-dimensional matrix, using a plurality of kernels can be interpreted as adding a further dimension (denoted as "depth" dimension), so that the nodes 29 of the convolutional layer 24 are arranged as a (d+1)-dimensional matrix. If the nodes 28 of the preceding layer 23 are already arranged as a (d+1)-dimensional matrix comprising a depth dimension, using a plurality of kernels can be interpreted as expanding along the depth dimension, so that the nodes 29 of the convolutional layer 64 are arranged also as a (d+1)-dimensional matrix, wherein the size of the (d+1)-dimensional matrix with respect to the depth dimension is by a factor of the number of kernels larger than in the preceding layer 23.

The advantage of using convolutional layers 24 is that spatially local correlation of the input data can exploited by enforcing a local connectivity pattern between nodes of adjacent layers, in particular by each node being connected to only a small region of the nodes of the preceding layer.

In the displayed embodiment, the input layer 23 comprises 36 nodes 28, arranged as a two-dimensional 6x6 matrix. The convolutional layer 24 comprises 72 nodes 29, arranged as two two-dimensional 6x6 matrices, each of the two matrices being the result of a convolution of the values of the input layer 23 with a kernel. Equivalently, the nodes 29 of the convolutional layer 24 can be interpreted as arranged as a three-dimensional 6x6x2 matrix, wherein the last dimension is the depth dimension.

A pooling layer 25 can be characterized by the structure and the weights of the incoming edges and the activation function of its nodes 30 forming a pooling operation based on a nonlinear pooling function f. For example, in the two-dimensional case the values $x^{(n)}$ of the nodes 30 of the pooling layer 25 can be calculated based on the values $x^{(n-1)}$ of the nodes 29 of the preceding layer 24 as

$$x^{(n)}[i,j] = f(x^{(n-1)}[id_1, jd_2], ..., x^{(n-1)}[id_1+d_1-1, jd_2+d_2-1])$$

In other words, by using a pooling layer 25 the number of nodes 29, 30 can be reduced, by replacing a number $d_1 \cdot d_2$ of neighboring nodes 29 in the preceding layer 24 with a single node 30 being calculated as a function of the values of said number of neighboring nodes in the pooling layer 25. In particular, the pooling function f can be the max-function, the average or the L2-Norm. In particular, for a pooling layer 25 the weights of the incoming edges are fixed and are not modified by training.

The advantage of using a pooling layer 25 is that the number of nodes 29, 30 and the number of parameters is reduced. This leads to the amount of computation in the network 22 being reduced and to a control of overfitting.

In the displayed embodiment, the pooling layer 25 is a max-pooling, replacing four neighboring nodes with only one node, the value being the maximum of the values of the four neighboring nodes. The max-pooling is applied to each d-dimensional matrix of the previous layer 24; in this embodiment, the max-pooling is applied to each of the two two-dimensional matrices, reducing the number of nodes from 72 to 18.

A fully-connected layer 26 can be characterized by the fact that a majority, in particular, all edges between nodes 30 of the previous layer 25 and the nodes 31 of the fully-connected layer 26 are present, and wherein the weight of each of the edges can be adjusted individually.

In this embodiment, the nodes 30 of the preceding layer 25 of the fully-connected layer 26 are displayed both as two-dimensional matrices, and additionally as non-related nodes (indicated as a line of nodes, wherein the number of nodes was reduced for a better presentability). In this embodiment, the number of nodes 31 in the fully connected layer 26 is equal to the number of nodes 30 in the preceding layer 25. Alternatively, the number of nodes 30, 31 can differ.

Furthermore, in this embodiment the values of the nodes 32 of the output layer 27 are determined by applying the Softmax function onto the values of the nodes 31 of the preceding layer 26. By applying the Softmax function, the sum of the values of all nodes 32 of the output layer 27 is 1, and all values of all nodes 32 of the output layer 27 are real numbers between 0 and 1. In particular, if using the convolutional neural network 22 for categorizing input data, the values of the output layer can be interpreted as the probability of the input data falling into one of the different categories.

A convolutional neural network 22 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are $f(x) = max(0,x)$, the tangent hyperbolics function or the sigmoid function.

In particular, convolutional neural networks 22 can be trained based on the backpropagation algorithm. For preventing overfitting, methods of regularization can be used, e.g. dropout of nodes 28 - 32, stochastic pooling, use of artificial data, weight decay based on the L1 or the L2 norm, or max norm constraints.

Fig. 3 is a flowchart of general embodiment of method according to the invention. In this example, a binary classifier comprising a CNN 22 is used as a trained first function.

Hence, in a step S1, first input data describing a case to be assessed is received. The first input data comprises image data of a patient, for example magnetic resonance image data, computed tomography image data, ultrasound image data, and the like. The image data may be two- and/or three-dimensional. The trained first function, in a step S2, analyses the image data (and optionally further input data) regarding learned patterns. The analysis yields first output data, which may, for example, be a list of at least one feature, for example lesions, with an associated confidence value indicating the probability of the feature being an (in particular certain) pathology. The list also comprises the respective locations of the features. The confidence value is associated with a result information, that is, a result information may be determined depending in the confidence value. In the exemplarily discussed binary classifier, for example, a feature may be found positive if a certain threshold is surpassed by the confidence value. In other embodiments, wherein multiple classes may result, additional confidence values may be determined and the classification result information may be determined by combining these confidence values. Generally, confidence values may also be called confidence scores or classification scores, in particular if they directly relate to the probability of a classification. It is noted that, due to the following potential modification of the confidence values, result information needs not be actually determined at this point.

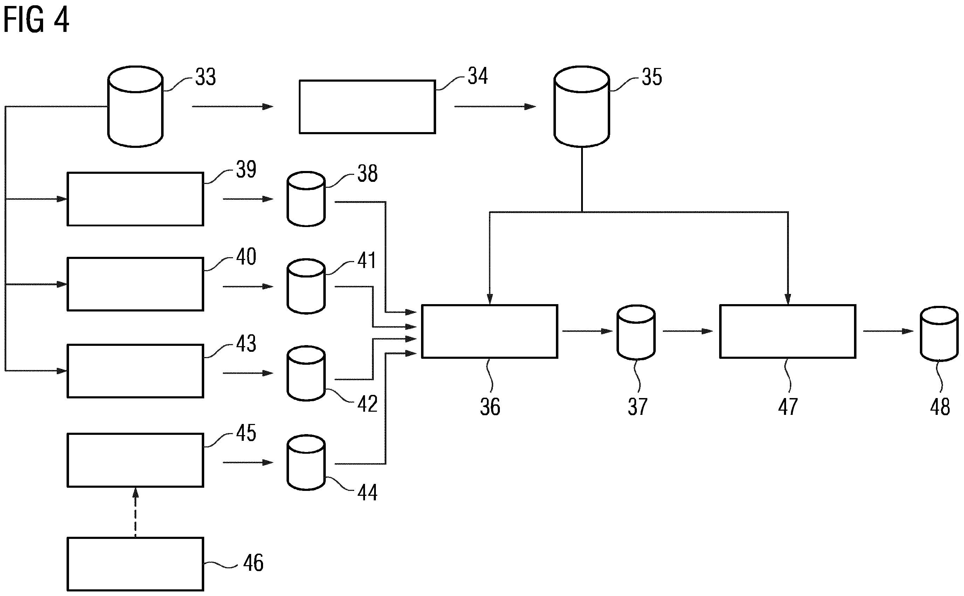

In a step S3, which may at least partly also be performed before steps S1 and S2 and/or parallel to them, case-specific background information is compiled. This case-specific background information, in addition to at least a part of the first output data, is used as second input data to a second function as further discussed below. The case-specific background information is compiled from different sources, as further shown with respect to Fig. 4.

Fig. 4 illustrates schematically the data flow and data processing in the method. As can be seen, the first input data 33 is used as input to the trained first function 34, which can be considered a fixed, pre-trained model, where training was performed unconditioned regarding the case-specific background information 37 to be compiled by compiling algorithm 36. Compiling algorithm 36 may also use the first output data 35 output from the trained first function 34.

As mentioned, multiple optional sources for case-specific background information 37 may be used. Anatomical information 38, which may be used as case-specific background information, may be provided at least partly as a result of at least one, particular trained, third function 39, which performs image processing on the image data of the first input data 33. The at least one third function 39 may, for example, comprise a segmentation function yielding segmentation information as anatomical information 38, particular segmented image data comprising labelled segments. At least one further, in particular trained, third function 40 may be used to determine shape information 41, in particular regarding at least one of the features of the list in the first output data 35. Since, as mentioned above, the anatomical location of the feature is also provided in the first output data 35, the correct feature for analysis and determination of geometrical shape may easily be found in the image data, which is also used here. It is noted that the third function 40 is an example for deriving case-specific background information 37, in this case the shape information 41, using first output data 35, in this case to identify the feature of interest.

Further source for background information 37, in this case metadata information 42, is a metadata selector 43, which selects relevant metadata to be used as case-specific background information from or associated with the first input data 33, particular from metadata associated with the image data. In particular, the metadata may be stored together with the image data in an image data file, for example in the DICOM standard. Metadata relevant as case-specific background information for modifying confidence values may comprise image acquisition parameters and/or image reconstruction parameters, for example slice thicknesses and the like. Furthermore, metadata concerning the patient, in particular demographically relevant metadata, may be selected by metadata selector 43, since some trained first functions 34 may be less reliable for some groups of patients and/or some pathologies may be less or more probable in some groups of patients.

Furthermore, additional clinical information 44 may be provided by the query module 45. The query module 45 may, for example, request additional clinical information from an information system 46, in particular an HIS or an RIS. In particular, the additional clinical information 44 may be extracted from an electronic patient record and/or patient registration data in the information system 46. Additional clinical information 44 relevant as case-specific background data may, for example, comprise laboratory results, physiological parameters of the patient (like heart rate, blood pressure and/or body temperature), inflammatory markers, risk scores and/or a clinical score (for example an already determined Glasgow Coma Scale score and/or anamnesis information of the patient. Anamnesis information may, for example, comprise information on prior surgery, implanted devices and the like.

The compiling algorithm 36 compiles the anatomical information 38, the shape information 41, the metadata information 42, and/or the additional clinical information 44, as well as optional further information, to yield the case-specific background information. Further information may (if not already included in any of information 38, 41, 42 or 44), for example, also comprise the classification of the case, in particular as as an edge case and/or as relating to a weakness of the trained first function 34. Such classification may, generally speaking, generally be derived from information 38, 41, 42 and 44. Such a derivation may, of course, also be implemented in the second function 47.

Returning to fig. 3, in step S4, the second function 47 is applied to the case-specific background information 37 and at least a part, comprising the confidence value, of the first output data 35 as second input data, to determine necessary modifications of the at least one confidence value, i.e., necessary conditioning of the at least one confidence value based on the case-specific background information 37. The second function 47 may comprise at least one non-AI algorithm and/or at least one trained conditioning function. In other words, in the conditioning model implemented as the second function 47, both engineered features based on clinical knowledge (without the use of artificial intelligence (AI)) as well as low-dimensional machine-learning based sub-models, for example regression, support vector machines and random forests, may be included. Depending on the implementation of the conditioning model, the output may directly be the (potentially modified) confidence values. In other implementations, a parametric monotonic confidence value calibration function, for example integral splines, may be determined, ub particular parametrized, and applied to determine the potentially modified confidence values.

In other words, the confidence values determined by the trained first function 34 are additionally conditioned, in particular based on prior knowledge, without the requirement to change or modify the trained first function 34. Prior knowledge, encoded in the second function, is applied to the current case, as described by the case-specific background information. For example, if an imaging technique used may result in information loss, the confidence value may be lowered. There may, of course, be (in particular many) cases in which the confidence value is not changed at all (for example for cases being "usual" and completely comparable to the ones used for training the first function). Cases, for which the confidence value is increased, are also possible, for example when patient metadata indicates that the patient belongs to a group with higher predisposition for a certain pathology and the like.

It is noted that modifying confidence values may also lead to different result information. Preferably, result information to be output in step S5 will hence be calculated based on the (potentially modified) confidence values, which are determined as second output data 48 (see fig. 4). In step S5, in addition to result information, also the potentially modified confidence values are output. Based on the output, a user, for example reading physician, can make supported clinical decisions.

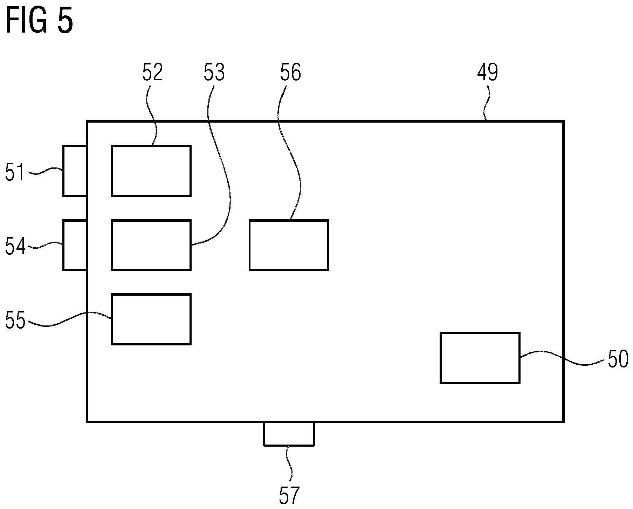

Fig. 5 shows the functional structure of a clinical decision support system 49 according to the invention. The clinical decision support system 49 comprises at least one memory 50, wherein all kinds of data used in the method can be at least temporarily stored.

The clinical decision support system 49 further comprises a first interface 51 for receiving the first input data 33 (step S1) and earn analysis unit 52 for applying the trained first function 34. A compilation unit 53 is adapted to gather and prepare the case-specific background information 37 according to step S3. Here, the compilation unit 53 may, in particular, access a second interface 54, which may, for example, be connected to the information system 46 or other sources of case-specific background information and/or basic information required to determine case-specific background information. The compilation unit 53 may also use at least one determination unit 55, which may implement the at least one, in particular trained, third function 39, 40. In this modification unit 56, the second function 47 is applied to determine potentially modified confidence values according to step S4. The potentially modified confidence values and result information may be output via output interface 57 according to step S5.

Although the present invention has been described in detail with reference to the preferred embodiment, the present invention is not limited by the disclosed examples from which the skilled person is able to derive other variations without departing from the scope of the invention.

Independent of the grammatical term usage, individuals with male, female or other gender identities are included within the term.

**Claims**

1. Computer-implemented method for clinical decision support, wherein a trained first function (34) determines first output data (35) regarding at least one clinical condition of a patient from first input data (33) comprising image data of the patient, the first output data (35) comprising at least one confidence value associated with or as result information, **characterized in that** the confidence value is modified by a second function (47) based on at least one case-specific background information (37) regarding the patient and/or the acquisition of the image data, the case-specific background information (37) provided as second input data to the second function (47).

2. Method according to claim 1, **characterized in that** the trained first function (34) is fixed and/or pre-trained unconditioned regarding the at least one case-specific background information (37).

3. Method according to claim 1 or 2, **characterized in that** the at least one case-specific background information (37) is at least partly derived from first output data (35), in particular regarding a feature, in particular a lesion, in the image data.

4. Method according to one of the preceding claims, **characterized in that** the case-specific background information (37) is chosen from the group comprising anatomical information (38), in particular an anatomical segmentation of the image data; shape information (41) describing the geometrical shape of a feature, in particular a lesion, comprised in the first output data (35); metadata information (42) associated with the first input data (33); and additional clinical information (44) regarding the patient.

5. Method according to claim 4, **characterized in that** the metadata information (42) comprises metadata relating to the patient and/or metadata relating to the acquisition of the image data.

6. Method according to claim 4 or 5, **characterized in that** the additional clinical information (44) is chosen from the group comprising at least one laboratory result; at least one physiological parameter of the patient; the presence of at least one inflammatory marker; at least one score, in particular a risk score and/or a clinical score, determined for the patient; and anamnesis information of the patient.

7. Method according to any of the claims 4 to 6, **characterized in that** the additional clinical information (44), in particular the anamnesis information, is at least partly determined from an electronic patient record of the patient and/or at least one patient registration information.

8. Method according to one of the preceding claims, **characterized in that** the case-specific background information (37) is at least partly provided by at least one trained third function (39, 40) using, as third input data, at least a part of the first input data (33), in particular the image data.

9. Method according to one of the preceding claims, **characterized in that** case-specific background information (37) comprises at least one classification of the case based on development and/or producer-internal evaluation information of the trained first function (34).

10. Method according to one of the preceding claims, **characterized in that** the second function (47) comprises a trained conditioning function.

11. Method according to claim 10, **characterized in that** the trained conditioning function has a lower dimensionality as the trained first function (34) and/or comprises a support vector machine and/or a random forest and/or a regression model.

12. Method according to one of the preceding claims, **characterized in that** the second function (47) comprises

    - determining parameters parametrizing a functional correlation between the confidence value of the first output data (35) and the modified confidence value, and
    - determining the modified confidence value by applying the functional correlation parametrized by the parameters to the confidence value of the first output data (35).

13. Clinical decision support system (49), comprising

    - a first interface (51) for receiving first input data (33), the first input data (33) comprising image data of a patient,
    - an analysis unit (52) for applying a trained first function (34) to the first input data (33) to determine first output data (35) regarding at least one clinical condition of a patient, the first output data (35) comprising at least one confidence value associated with or as result information,
    - a compilation unit (53) for compiling case-specific background information (37) regarding the patient and/or the acquisition of the image data,
    - a modification unit (56) for modifying the confidence value by a second function (47) based on at least one case-specific background information (37), the at least one case-specific background information (37) being provided as second input data to the second function (47), and

- an output interface (57) for outputting the modified confidence value.

14. Computer program, which, when executed in a clinical decision support system, causes the clinical decision support system (49) to perform the steps of a method according to one of the claims 1 to 12.

15. Electronically readable storage medium, on which a computer program according to claim 14 is stored.

FIG 1

**FIG 2**

FIG 3

S1

S2

S3

S4

S5



# FIG 4

EP 4 517 768 A1

## FIG 5

**EP 4 517 768 A1**

## EUROPEAN SEARCH REPORT

Application Number

EP 23 19 5163

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/004863 A1 (PARK SUN YOUNG [US] ET AL) 6 January 2022 (2022-01-06) * paragraph [0032] - paragraph [0037]; figure 2 * * paragraph [0045] - paragraph [0055]; figures 4,5,6 * | 1-15 | INV. G16H30/00 G06T7/00 G16H50/20 |
| X | MEHTA RAGHAV ET AL: "Propagating Uncertainty Across Cascaded Medical Imaging Tasks for Improved Deep Learning Inference", IEEE TRANSACTIONS ON MEDICAL IMAGING, vol. 41, no. 2, 1 February 2022 (2022-02-01), pages 360-373, XP093123011, USA ISSN: 0278-0062, DOI: 10.1109/TMI.2021.3114097 Retrieved from the Internet: URL:https://ieeexplore.ieee.org/stampPDF/g etPDF.jsp?tp=&arnumber=9541203&ref=aHR0cHM 6Ly9pZWVleHBsb3JlLmllZWUub3JnL2Fic3RyYWN0L2RvY3VtZW50Lzk1NDEyMDM=> * Chapter "II. METHODOLOGY: PROPAGATING UNCERTAINTY ACROSS INFERENCE TASKS"; figure 1 * | 1-15 | |
| X | KR 2022 0159085 A (VUNO INC [KR]; UNIV KOREA RES & BUS FOUND [KR]) 2 December 2022 (2022-12-02) * paragraph [0065] - paragraph [0107] * | 1-15 | TECHNICAL FIELDS SEARCHED (IPC) G16H G06T |

-/--

The present search report has been drawn up for all claims

| Place of search | Date of completion of the search | Examiner |
|---|---|---|
| Munich | 2 February 2024 | Eichenauer, Lars |

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WENHAO LI ET AL: "Interactive Medical Image Segmentation with Self-Adaptive Confidence Calibration", ARXIV.ORG, CORNELL UNIVERSITY LIBRARY, 201 OLIN LIBRARY CORNELL UNIVERSITY ITHACA, NY 14853, 15 November 2021 (2021-11-15), XP091098874, * abstract & chapters 1 and 3 * | 1-15 | |
| A | US 2021/398655 A1 (EVANS NEIL REZA SHADBEH [US] ET AL) 23 December 2021 (2021-12-23) * paragraph [0073] – paragraph [0078] * | 1-15 | |
| A | Anonymous: "Functional correlation", Wikipedia, 21 February 2023 (2023-02-21), pages 1-3, XP093126981, Retrieved from the Internet: URL:https://en.wikipedia.org/w/index.php?title=Functional_correlation&oldid=1140782740 [retrieved on 2024-02-02] * the whole document * | 12 | |
| | | | **TECHNICAL FIELDS SEARCHED (IPC)** |

The present search report has been drawn up for all claims

| Place of search | Date of completion of the search | Examiner |
|---|---|---|
| Munich | 2 February 2024 | Eichenauer, Lars |

EPO FORM 1503 03.82 (P04C01)

## ANNEX TO THE EUROPEAN SEARCH REPORT
## ON EUROPEAN PATENT APPLICATION NO.

EP 23 19 5163

This annex lists the patent family members relating to the patent documents cited in the above-mentioned European search report.
The members are as contained in the European Patent Office EDP file on
The European Patent Office is in no way liable for these particulars which are merely given for the purpose of information.

02-02-2024

| Patent document cited in search report | Publication date | Patent family member(s) | | Publication date |
|---|---|---|---|---|
| US 2022004863 A1 | 06-01-2022 | NONE | | |
| KR 20220159085 A | 02-12-2022 | KR 20220159085 A | | 02-12-2022 |
| | | KR 20230151962 A | | 02-11-2023 |
| US 2021398655 A1 | 23-12-2021 | US 2021398655 A1 | | 23-12-2021 |
| | | US 2021398676 A1 | | 23-12-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20200320354 A1
- US 20220293247 A1

- US 20220309667 A1

**Non-patent literature cited in the description**

- **WEIJIE CHEN et al.** Calibration of medical diagnostic classifier scores to the probability of disease. *Stat Methods Med Res.*, 2018, vol. 27, 1394-1409